# FASCICULE DE BREVET EUROPEEN

(11) **EP 1 698 305 B1**
(45) Date de publication et mention de la délivrance du brevet: **08.08.2007**
(21) Numéro de dépôt: 05108139.6
(22) Date de dépôt: 06.09.2005
(51) Int. Cl.: A61F 2/44, A61F 2/46

(54) **Prothèse déformable d'écartement de corps vertébraux**
Verformbare Zwischenwirbelprothese
Deformable intervertebral prosthesis

(43) Date de publication de la demande: 06.09.2006
(73) Titulaire: ORTHOPAEDIC & SPINE DEVELOPMENT, 84911 Avignon Cédex 9 (FR)
(72) Inventeur: Nemoz-Gaillard, Isabelle, 30340, Mons (FR)
(74) Mandataire: Schmitz, Jean-Marie

(56) Documents cités:
- EP-A- 1 138 285
- DE-C1- 4 416 605
- FR-A- 2 803 741
- FR-A- 2 843 017
- US-B1- 6 454 807
- US-B1- 6 685 742

## Description

La présente invention concerne une prothèse déformable d'écartement de corps vertébraux décrite selon la revendication 1.

Le domaine technique auquel se rapporte cette invention concerne le domaine chirurgical, notamment le traitement d'individus souffrant d'une lordose (courbure de la colonne vertébrale à convexité antérieure caractérisé par un creux lombaire engendrant une bascule du bassin vers l'avant) et/ou d'une cyphose au niveau des vertèbres cervicales (déviation de la colonne vertébrale à convexité postérieure).

L'Etat de la technique est constitué par le brevet européen EP 1 532 949 et le brevet français FR2753368 concernant une cage d'ostéosynthèse expansive constituée d'une partie male coopérant avec une partie femelle, l'élément male est constitué de deux parties appelées cale et bouchon, ledit élément male étant introduit selon un axe longitudinal horizontal à l'intérieur de l'élément femelle. Le brevet français FR 2803741 décrit une cage intersomatique comprenant un élément femelle constitué par une glissière en forme de V et un élément male, l'élément male étant introduit selon un axe longitudinal horizontal. Le brevet européen EP 1290985 concerne une cage intersomatique ayant une partie supérieure et une partie inférieure ajourée et munis de crans.

DE-44 16 605 décrit un implant inter-vertébral selon le préambule de la revendication 1, ayant un poussoir se vissant selon un axe horizontal par rapport à l'implant, cet axe correspondant à l'axe de symétrie horizontal de l' implant.

EP 1 138 285 concerne une cage vertébrale insérée entres les vertèbres de la colonne vertébrale.

US-A-6 454 807 divulgue un système de cage de fusion vertébrale expansible et articulé.

FR- 28 43 017 décrit un implant inter-somatique.

Les prothèses de l'état de la technique ont des dimensions et des formes géométriques déterminées qui sont des approximations des dimensions et des formes géométriques des corps vertébraux entre lesquels les prothèses sont destinées à être introduites, sans toutefois être parfaitement complémentaires. C'est pourquoi il est nécessaire de conformer les surfaces des plateaux des corps vertébraux pour qu'elles soient le plus complémentaire possible des surfaces correspondantes de la prothèse choisie.

La **différence** entre l'état de la technique et la présente invention est que l'axe longitudinal de ladite ouverture (8) et du poussoir de réglage (2) est incliné par rapport à l'axe longitudinal central du corps de prothèse (1), les deux axes restant généralement dans un plan sagittal du corps de prothèse (1) (plan sagittal = plan vertical de symétrie). La translation du poussoir de réglage peut se faire selon un axe incliné de 1° à 15° par rapport à l'axe longitudinal, de préférence de 1° à 10°. Aucun art antérieur ne décrit cette translation selon un axe incliné.

**L'effet technique** atteint par cette différence est que l'on obtient une inclinaison de la partie supérieure (3) de la prothèse, ladite partie supérieure de la prothèse épousant ainsi la forme des corps vertébraux de manière optimale corrigeant de façon surprenante et très efficace l'angle de lordose et/ou d'une cyphose entre deux vertèbres. La partie inférieure (6) plate de la prothèse épouse aussi la forme des corps vertébraux de manière optimale.

La présente invention a pour but de proposer une prothèse qui soit de conception simple de sorte à être mise facilement en oeuvre et qui épouse le plus parfaitement possible la forme des corps vertébraux inférieurs et supérieurs de sorte à rectifier de manière optimale une lordose et/ou une cyphose et ainsi d'améliorer le traitement chirurgical.

L'invention est décrite ci-après à l'aide de références aux dessins ci-joints dans lesquels :

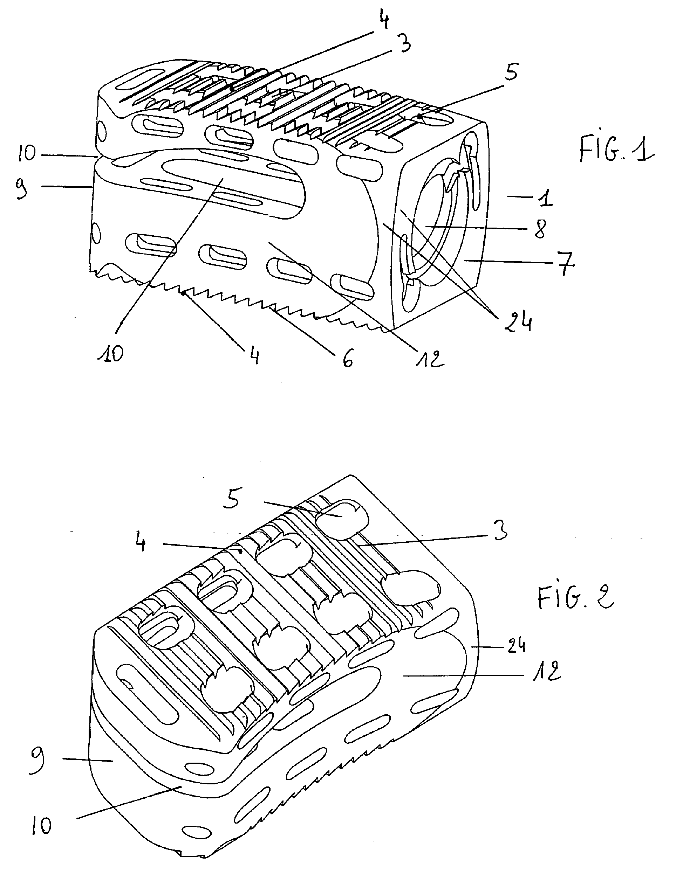

La figure 1 représente une vue en perspective de la partie postérieure (7), de la paroi latérale longitudinale concave (12) et du partie supérieure (3) du corps de prothèse (1) de la prothèse déformable d'écartement de corps vertébraux.
La figure 2 représente une vue en perspective de la partie supérieure (3), de la partie antérieure (9) et de la paroi latérale longitudinale concave (12) du corps de prothèse déformable d'écartement de corps vertébraux.

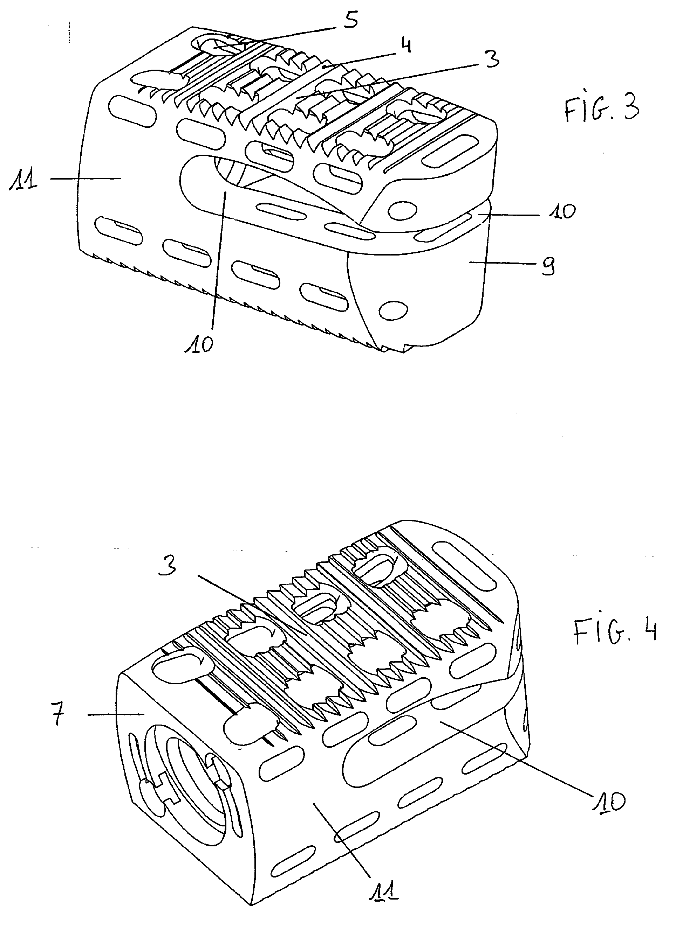

La figure 3 représente une vue en perspective de la partie antérieure (9), de la partie supérieure (3) et de la paroi latérale longitudinale plate (11) du corps de prothèse déformable d'écartement de corps vertébraux.
La figure 4 représente une vue en perspective de la partie postérieure (7), de la paroi latérale longitudinale plate (11) et de la partie supérieure (3) du corps de prothèse (1) déformable d'écartement de corps vertébraux.

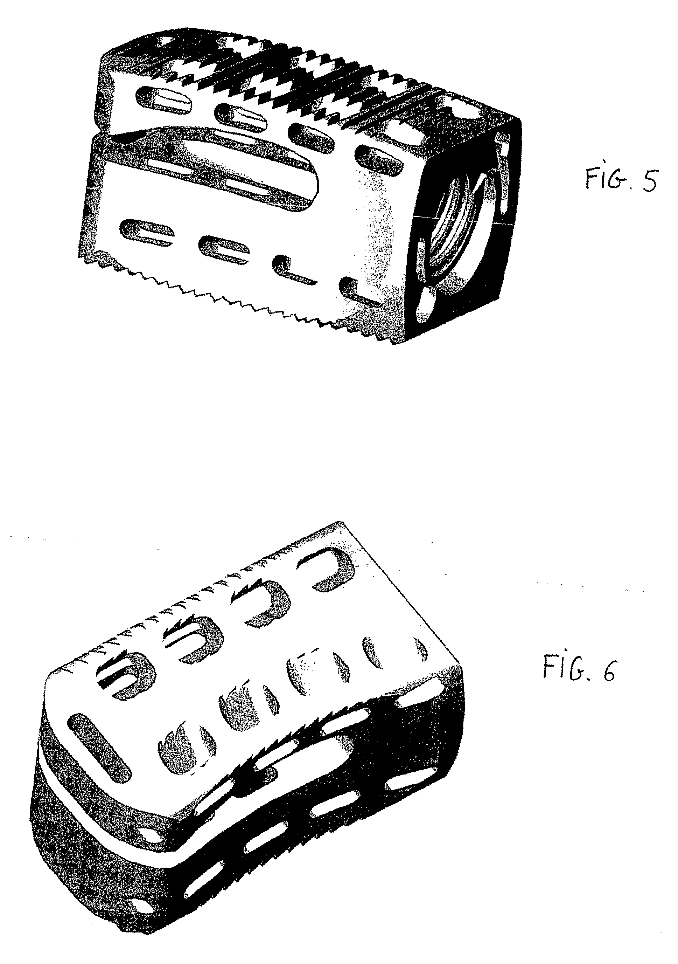

La figure 5 représente une photographie du corps de prothèse déformable d'écartement de corps vertébraux de la figure 1.
La figure 6 représente une photographie du corps de prothèse déformable d'écartement de corps vertébraux de la figure 2.

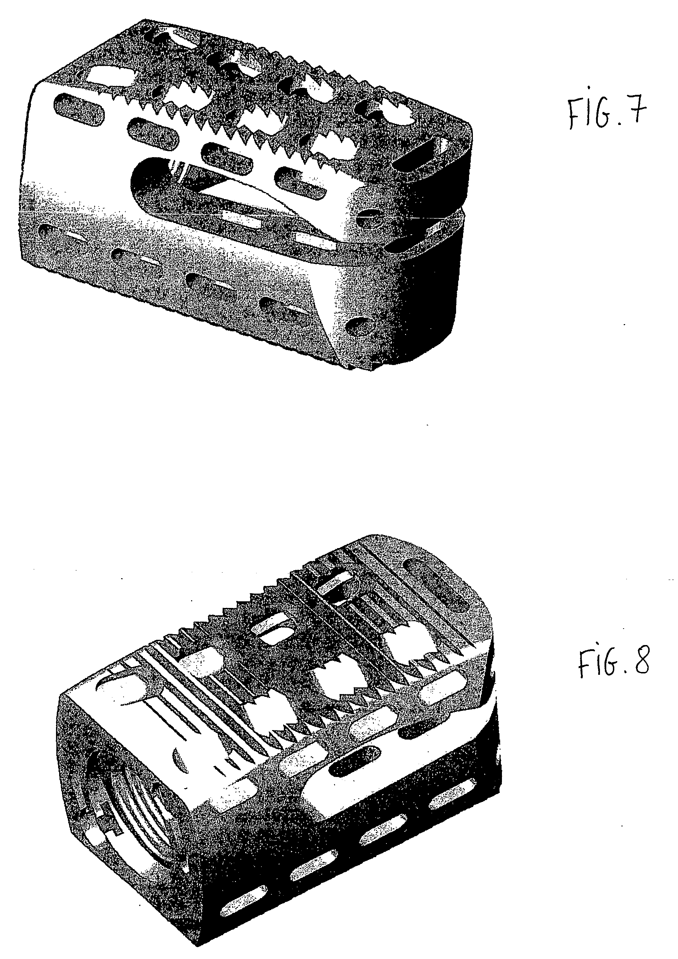

La figure 7 représente une photographie du corps de prothèse déformable d'écartement de corps vertébraux de la figure 3.
La figure 8 représente une photographie du corps de prothèse déformable d'écartement de corps vertébraux de la figure 4.

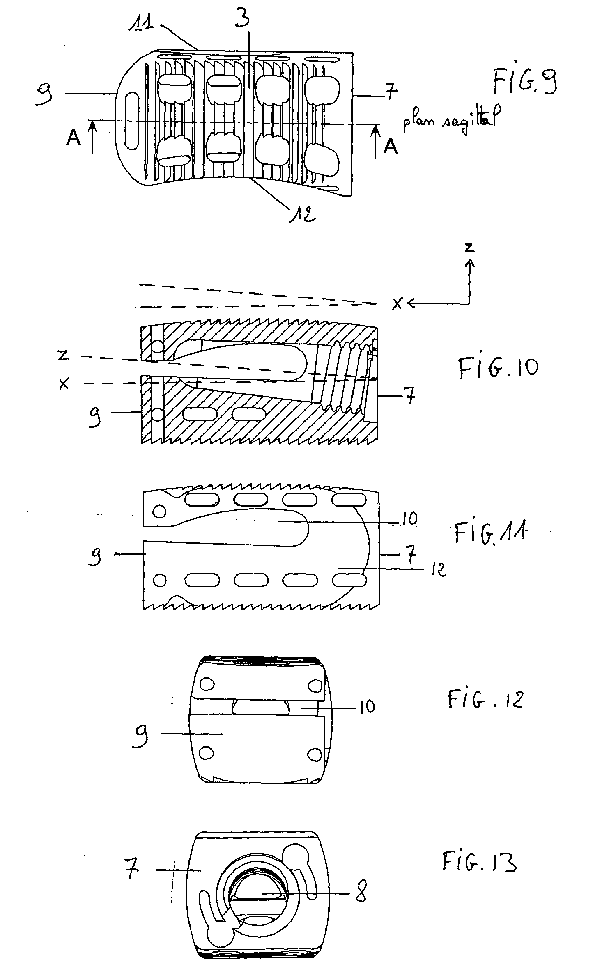

La figure 9 représente une vue de la partie supérieure (3) du corps de prothèse déformable d'écartement de corps vertébraux et l'axe A-A correspond au plan sagittal.
La figure 10 représente une coupe en section suivant l'axe A-A (plan sagittal) du corps de prothèse déformable d'écartement de corps vertébraux, l'axe X correspondant à l'axe longitudinal de la prothèse et l'axe Z correspondant à l'axe de translation du poussoir de réglage. La translation du poussoir de réglage (2) à l'intérieur du corps de prothèse (1) se faisant selon un axe incliné Z par rapport à l'axe longitudinal, les deux axes restant dans le plan sagittal.

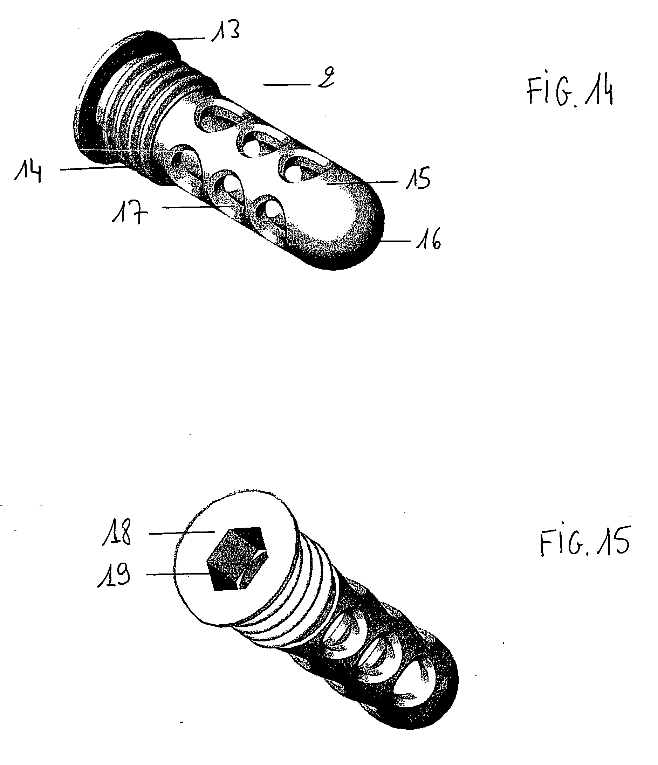

La figure 11 représente une vue de la paroi latérale longitudinale concave (12) et de la fente (10).
La figure 12 représente une vue de la partie antérieure du corps de prothèse (1) déformable d'écartement de corps vertébraux.

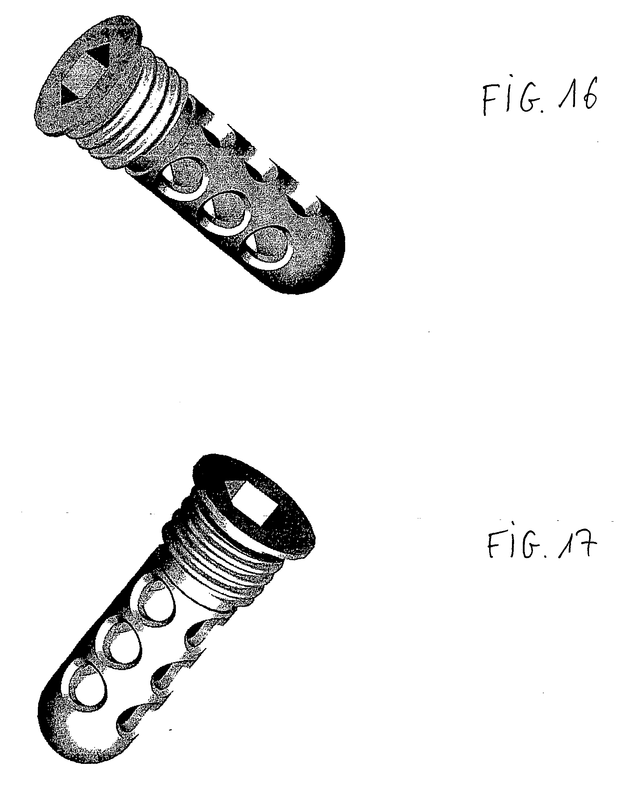

La figure 13 représente une vue de la partie postérieure (7) du corps de prothèse déformable d'écartement de corps vertébraux ainsi que deux orifices permettant de recevoir l'ancillaire et le maintien de l'ensemble ancillaire -poussoir de réglage -corps de prothèse.
La figure 14 représente une vue en perspective du poussoir de réglage (2) allongé servant de poussoir et comprenant une première partie (13) servant de butée, une deuxième partie (14) constituée par un pas de vis et une troisième partie (15) allongée perforée,
La figure 15 représente une vue en perspective du poussoir de réglage (2) allongé ayant une tête circulaire (18) et une ouverture centrale hexagonale (19) pour l'insertion de l'ancillaire (90) lors de la mise en place du poussoir de réglage (2) à l'intérieur du corps de prothèse (1).

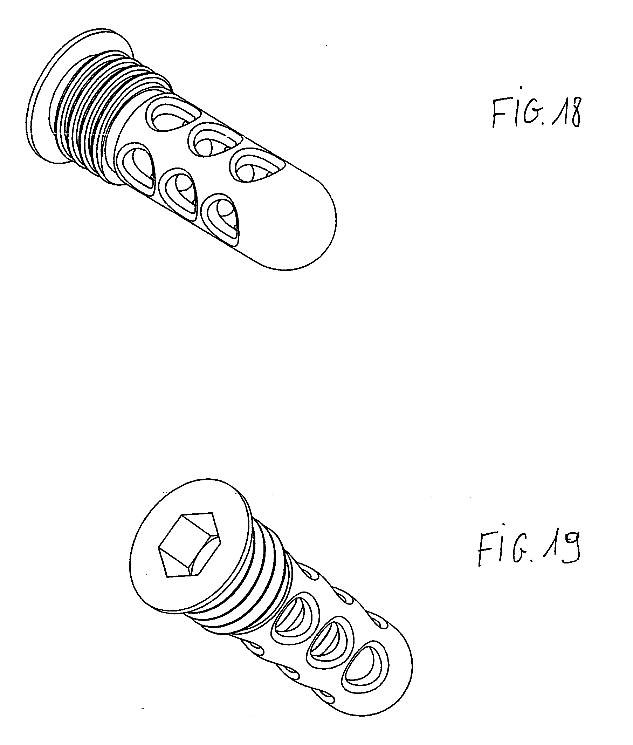

La figure 16 représente une autre vue en perspective du poussoir de réglage allongé.
La figure 17 représente encore une autre vue en perspective du poussoir de réglage allongé.
La figure 18 représente une vue schématique de la figure 14.

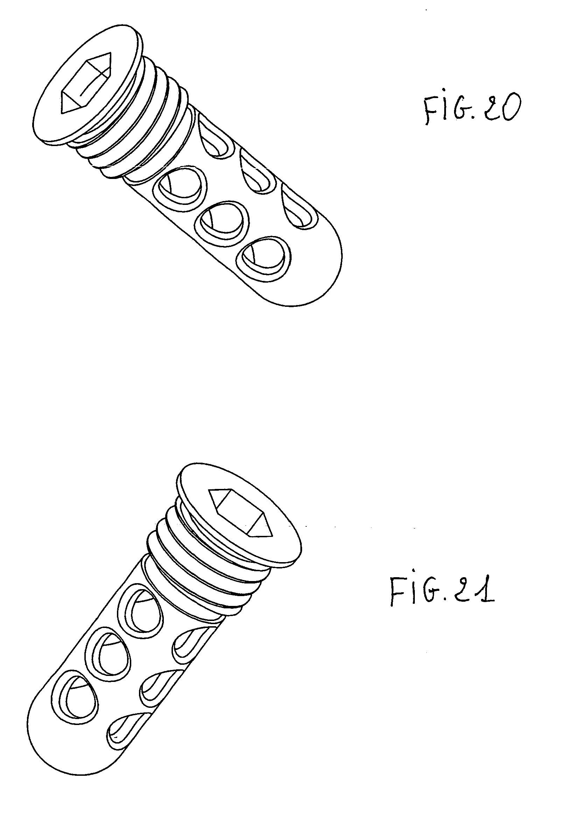

La figure 19 représente une vue schématique de la figure 15.
La figure 20 représente une vue schématique de la figure 16.
La figure 21 représente une vue schématique de la figure 17.

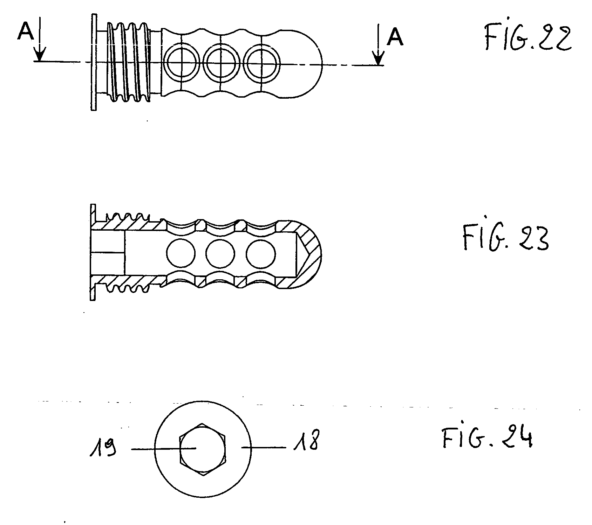

La figure 22 représente une vue schématique du poussoir de réglage et un axe A-A.
La figure 23 représente une vue schématique d'une coupe en section du poussoir de réglage suivant l'axe A-A.
La figure 24 représente une vue schématique de la tête (18) du poussoir de réglage.

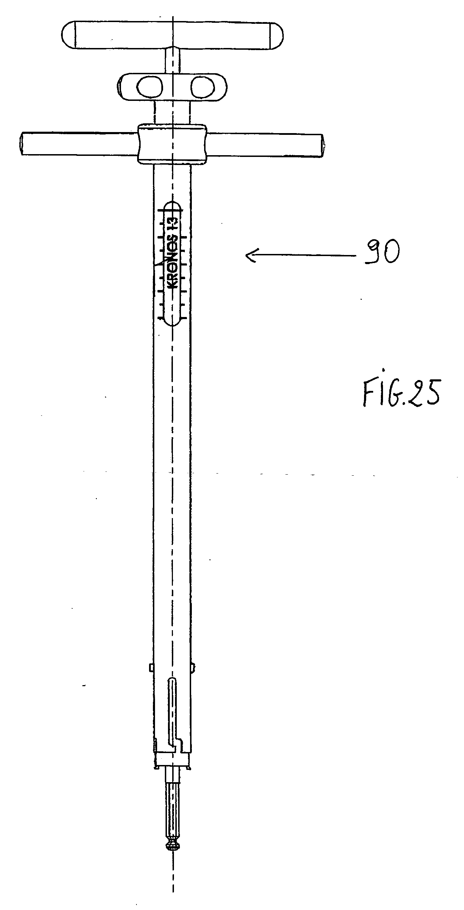

La figure 25 représente une vue schématique de l'ancillaire (90) permettant l'insertion du poussoir de réglage (2) à l'intérieur du corps de prothèse (1).

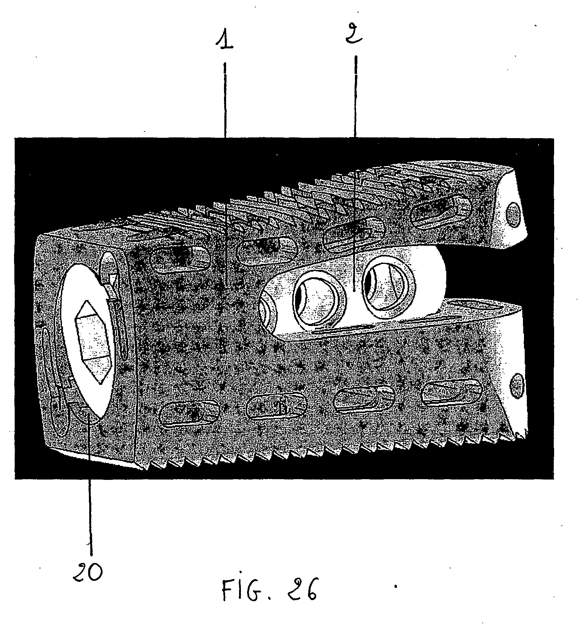

La figure 26 représente une vue en perspective de la prothèse déformable d'écartement de corps vertébraux constituée d'un corps de prothèse (1) et d'un poussoir de réglage (2) engagé dans ledit corps de prothèse (1).

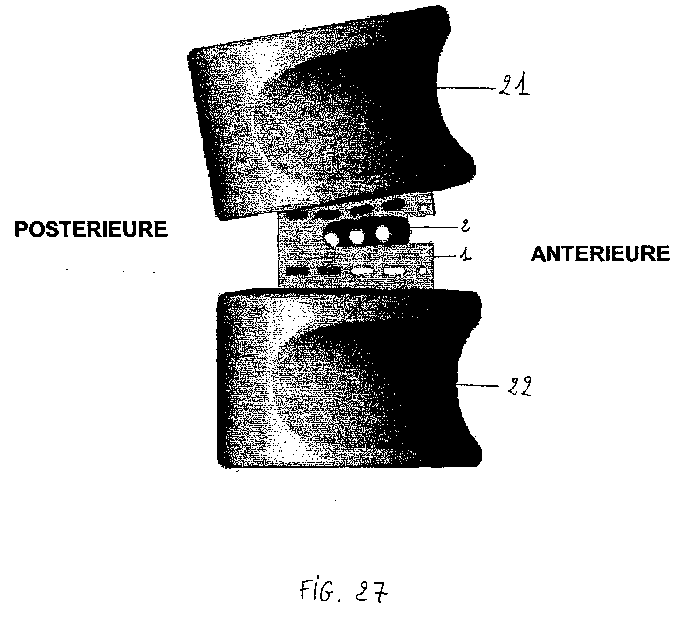

La figure 27 représente une prothèse déformable d'écartement de corps vertébraux en contact avec un corps vertébral supérieur (21) et inférieur (22).

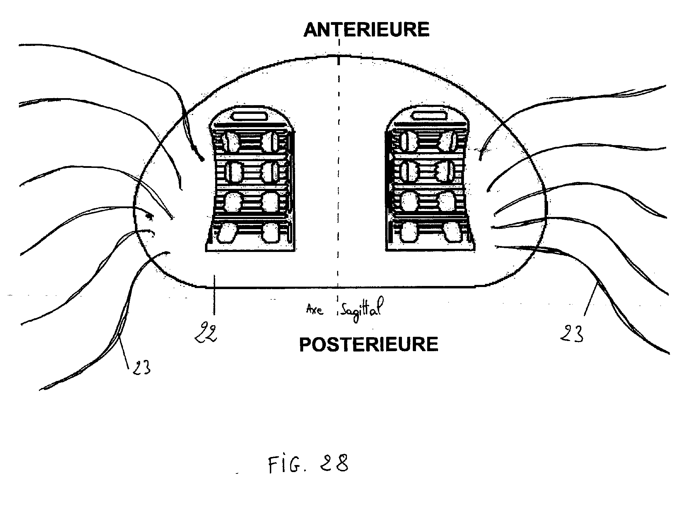

La figure 28 représente une prothèse droite et une prothèse gauche (par rapport à un plan sagittal) en contact avec un corps vertébral inférieur (22) d'où partent des racines périphériques (23).

Conformément à l'invention le but est atteint grâce à une prothèse déformable d'écartement de corps vertébraux constituée d'un corps de prothèse (1) ajouré pouvant être de type « nid d'abeille» et d'un poussoir de réglage (2) allongé coopérant avec ce corps de prothèse (1), ledit corps de prothèse (1) comprenant une partie supérieure (3) convexe comportant des crans transversaux (4) en forme de dents et des lumières (5) de communication osseuse dans le corps de prothèse et permettant le maintien en place de la prothèse, une partie inférieure (6) plate comportant des crans transversaux (4) en forme de dents et des lumières (5) de migration osseuse dans le corps de prothèse, une partie postérieure (7) plate comprenant une ouverture circulaire longitudinale (8) recevant le poussoir de réglage (2), ladite ouverture circulaire longitudinale (8) de la partie postérieure (7) est taraudée à sa partie antérieure, une partie antérieure (9) arrondie pourvue d'une fente (10) séparant la partie supérieure (3) de la partie inférieure (6), une paroi latérale (11) longitudinale plate coupée en deux parties par la fente (10), une paroi latérale (12) longitudinale concave opposée à la paroi latérale (11) longitudinale plate également coupée en deux par la fente (10), la translation du poussoir de réglage (2) à l'intérieur du corps de prothèse (1) se faisant selon un axe incliné par rapport à l'axe longitudinal, les deux axes restant dans le plan sagittal.
La partie supérieure (3) et la partie inférieure (6) sont solidaires l'une de l'autre sur une partie de la longueur du corps de prothèse (1) et sont espacées l'une de l'autre par une fente (10) s'étendant de la partie solidarisée et débouchant à l'une des extrémités du corps et traversant le corps de prothèse (1) sur toute sa largeur. La fente (10) s'étend sur au moins la moitié, de préférence les deux tiers de la longueur du corps de prothèse (1).

La partie supérieure et la partie inférieure sont munies de crans de façon à éviter tout mouvement relatif entre la prothèse (ou écarteur intersomatique) et les plateaux des corps vertébraux entre lesquels la prothèse est insérée (mouvement avant-arrière et gauche-droite). De façon avantageuse la prothèse est munie de lumières pour favoriser la fusion entre les vertèbres et la migration osseuse.

La translation du poussoir de réglage se fait selon un axe incliné de 1 ° à 15° par rapport à l'axe longitudinal, de préférence de 1° à 10°. Les lumières (5) de la partie supérieure (3) et de la partie inférieure (6) sont alignées longitudinalement.

La partie supérieure (3) de la prothèse est déformable et est en contact avec un corps vertébral supérieur (21). Cette partie supérieure (3) épouse la concavité du corps vertébral supérieur selon deux directions : dans le plan sagittal et dans le plan frontal.

La partie inférieure (6) de la prothèse est indéformable et est en contact avec un corps vertébral inférieur (22). La partie supérieure (3) et la partie inférieure (6) se retrouvent reliées par l'intermédiaire d'un pont dans la partie postérieure (7). La partie antérieure (9) et la partie postérieure (7) possèdent des arêtes et des extrémités non saillantes.

Le poussoir de réglage (2) allongé comprend une première partie (13) servant de butée, une deuxième partie (14) constituée par un pas de vis complémentaire à la partie taraudée de l'ouverture (8) de la partie postérieure du corps de prothèse et une troisième partie (15) allongée lisse qui peut présenter des lumières (17) traversantes possédant des bords arrondis lisses. Le poussoir de réglage (2) allongé est de forme cylindrique et possède un filetage de type male pour pouvoir être vissé dans le filetage de type femelle de l'ouverture de la partie postérieure plate. L'extrémité antérieure dudit poussoir de réglage (2) allongé peut être hémisphérique (16). Le poussoir de réglage (2) peut être creux afin de recevoir une croissance osseuse. Lorsque le poussoir de réglage (2) est entièrement à l'intérieur du corps de prothèse (1), la butée se trouvant à son extrémité postérieure est arrêtée par un épaulement (20) du corps de prothèse. La pénétration du poussoir de réglage (2) à l'intérieur du corps de prothèse (1) provoque la déformation par inclinaison de la partie supérieure (3). Le poussoir de réglage est muni de lumières (17) traversantes qui jouent le rôle de diffuseur du greffon pour optimiser l'ostéointégration.

La prothèse de la présente invention est une prothèse droite ou une prothèse gauche (symétrie miroir par rapport au plan sagittal) pour s'adapter à l'anatomie des corps vertébraux et protéger les racines périphériques (23). Ainsi une ou deux prothèses (suivant l'orientation donnée aux prothèses) peuvent être en contact avec un corps vertébral supérieur (21) et un corps vertébral inférieur (22).

Cette prothèse comporte six surfaces asymétriques. Les caractéristiques géométriques de la prothèse ont été spécialement et minutieusement choisies pour éviter tout endommagement de la dure-mère, des racines périphériques (23) et de tout tissu sensible.

La prothèse peut être réalisée en matière minérale ou synthétique par exemple en PEEK (poly ester ethyl carbon), en titane ou en tout autre matière acceptant les contraintes. La prothèse s'adapte à la morphologie par déformation tridimensionnelle (flexion et torsion) sous l'action simultanée d'un poussoir de réglage et des corps vertébraux. La déformation principale concerne la partie supérieure de la prothèse et permet d'obtenir un fort effet lordosant. La déformation de la prothèse permet de supprimer l'impaction de la prothèse dans les corps vertébraux.

L'insertion du poussoir de réglage se fait au moyen d'un ancillaire (dispositif de mise en place d'un écarteur de corps vertébraux) approprié comportant une poignée de préhension solidaire d'un tube, une tige suiveuse guidée et immobilisée à l'intérieur du tube et des moyens d'assemblage qui sont constitués d'une tige guidée à l'intérieur d'un premier alésage du tube, une molette permettant l'entraînement en rotation de la tige et d'un profil hexagonal prévu dans un second alésage dudit tube pour le blocage en rotation de la tige suiveuse à l'intérieur du tube.

## Revendications

1. Prothèse déformable d'écartement de corps vertébraux, constituée d'un **corps de prothèse** (1) et d'un **poussoir de réglage** (2) engagé dans ledit corps de prothèse (1), ce corps de prothèse (1) comportant
une **partie supérieure** (3) et une **partie inférieure** (6), ces deux parties étant solidaires l'une de l'autre sur une partie de la longueur du corps de prothèse (1) et étant espacées l'une de l'autre par une **fente** (10) s'étendant de la **partie solidarisée** (24) et débouchant à l'une des extrémités du corps et traversant le corps de prothèse (1) sur toute sa largeur,
le poussoir de réglage (2) étant engagé dans une **ouverture** (8) pratiquée dans ladite partie solidarisée (24) et s'étendant dans ladite fente (10), la position axiale du poussoir de réglage (2) par rapport au corps de prothèse (1) étant réglable pour régler l'écartement entre les parties non solidarisées du corps de prothèse (1), **caractérisée en ce que** l'axe longitudinal de ladite ouverture (8) et du poussoir de réglage (2) est incliné par rapport à l'axe longitudinal central H est du corps de prothèse (1), les deux axes restant généralement dans un plan sagittal du corps de prothèse (1).

2. Prothèse selon la revendication 1, dans laquelle l'axe longitudinal de l'ouverture (8) et du poussoir de réglage (2) est incliné de 1 à 15° par rapport à l'axe longitudinal central du corps de prothèse (1) par rapport à l'axe longitudinal central du corps de prothèse, l'inclinaison étant de préférence de 1° à 10°.

3. Prothèse selon la revendication 1, comprenant une **paroi latérale** (11) longitudinale **plate** séparée en deux parties par la fente (10).

4. Prothèse selon la revendication 1, comprenant une **paroi latérale** (12) longitudinale **concave** opposée à la paroi latérale (11) longitudinale plate également séparée en deux par la fente (10).

5. Prothèse selon la revendication 1, comprenant une **partie postérieure** (7) plate.

6. Prothèse selon la revendication 1, comprenant une **partie antérieure** (9) arrondie.

7. Prothèse selon la revendication 1, dans laquelle ledit corps de prothèse (1) comporte une structure ajourée de type « nid d'abeille ».

8. Prothèse selon la revendication 1, dans laquelle la partie supérieure (3) et la partie inférieure (6) possèdent des lumières (5) alignées longitudinalement et des crans transversaux (4) en forme de dents.

9. Prothèse selon la revendication 6, dans laquelle la partie supérieure (3) est convexe et déformable et épouse la concavité du corps vertébral supérieur dans le plan sagittal et dans le plan frontal.

10. Prothèse selon la revendication 1, dans laquelle la fente (10) s'étend sur au moins la moitié de la longueur du corps de prothèse (1).

11. Prothèse selon la revendication 1, dans laquelle la fente (10) s'étend sur au moins les deux tiers de la longueur du corps de prothèse (1).

12. Prothèse selon la revendication 1, dans laquelle la partie solidarisée correspond à la partie postérieure (7) et est opposée à la partie antérieure (9).

13. Prothèse selon la revendication 1, dans laquelle la partie inférieure (6) est indéformable et plate.

14. Prothèse selon la revendication 1, dans laquelle ledit poussoir de réglage (2) est creux, de forme allongée cylindrique et comprend une première partie (13) servant de butée, une deuxième partie (14) constituée par un pas de vis et une troisième partie (15) lisse, allongée hémisphérique (16) et présentant des lumières (17) traversantes.

15. Prothèse selon la revendication 1, ladite prothèse étant une prothèse droite ou une prothèse gauche par rapport à un plan sagittal.

## Claims

1. Deformable prosthesis for spacing vertebral bodies, consisting of a prosthesis body (1) and an adjusting device (2) engaging into said prosthesis body (1), the prosthesis body (1) comprising an upper part (3) and a lower part (6), with said two parts being continuous over a part of the length of the prosthesis body (1) and being spaced from each other by means of a slot (10) extending from the continuous part (24) to one of the extremities of the body and traversing the prosthesis body (1) over its whole width,
wherein the adjusting device (2) is engaged in an opening (8) provided in said continuous part (24) and extends into said slot (10), with the axial position of the adjusting device (2) with respect to the prosthesis body (1) being adjustable for adjusting the distance between the non continuous parts of the prosthesis body (1),
**characterized in that** the longitudinal axis of said opening (8) and of the adjusting device (2) is inclined with respect to the central longitudinal axis of the prosthesis body (1), while the two axis generally remain on a sagittal plane of the prosthesis body (1).

2. Prosthesis according to claim 1, in which the longitudinal axis of said opening (8) and of the adjusting device (2) is inclined by about 1 to 15° with respect to the central longitudinal axis of the prosthesis body (1), wherein the preferred inclination is of from 1 ° to 10°.

3. Prosthesis according to claim 1, comprising a lateral longitudinal plane wall (11) that is separated in two parts by the slot (10).

4. Prosthesis according to claim 1, comprising a lateral longitudinal concave wall (12) that also is separated in two parts by the slot (10) and is situated opposite the lateral longitudinal plane wall (11).

5. Prosthesis according to claim 1, comprising a plane posterior part (7).

6. Prosthesis according to claim 1, comprising a rounded anterior part (9).

7. Prosthesis according to claim 1, in which said prosthesis body (1) comprises a structure which is perforated in a comb-like pattern.

8. Prosthesis according to claim 1, in which the upper part (3) and the lower part (6) are provided with longitudinally aligned openings (5) and with transverse channels (4) having the form of teeth.

9. Prosthesis according to claim 6, in which the upper part (3) is convex and deformable and adjacent to the concavity of the upper vertebral body on the sagittal level and on the frontal level.

10. Prosthesis according to claim 1, in which the slot (10) is extending over at least half of the length of the prosthesis body (1).

11. Prosthesis according to claim 1, in which the slot (10) is extending over at least two thirds of the length of the prosthesis body (1).

12. Prosthesis according to claim 1, in which the continuous part corresponds to the posterior part (7) and is situated opposite the anterior part (9).

13. Prosthesis according to claim 1, in which the lower part (6) can not be deformed and is plane.

14. Prosthesis according to claim 1, in which said adjusting device 2 is hollow, of extended, cylindrical form and comprises a first part (13) serving as stopper, a second part (14) constituted of a screw thread and a smooth third part (15) in form of a hemisphere (16) and provided with traversing openings (17).

15. Prosthesis according to claim 1, said prosthesis being a right prosthesis or a left prosthesis with respect to a sagittal plane.

## Patentansprüche

1. Verformbare Prothese zur Spreizung von Wirbelkörpern, bestehend aus einem Prothesenkörper (1) und einer Justierungsvorrichtung (2), die in den Prothesenkörper (1) hineinreicht,
wobei der Prothesenkörper (1) einen oberen Teil (3) und einen unteren Teil (6) aufweist und diese beiden Teile über einen Teil der Länge des Porthesenkörpers (1) durchgehend und voneinander durch einen Spalt (10) beabstandet sind, der sich von dem durchgehenden Teil (24) bis zu einem Ende des Körpers erstreckt und den Prothesenkörper (1) auf seiner gesamten Breite durchmisst,
wobei die Justierungsvorrichtung (2), die in eine in dem durchgehenden Teil (24) vorgesehene Öffnung (8) hineinreicht und sich in den Spalt (10) erstreckt, wobei die axiale Position der Justierungsvorrichtung (2) mit Bezug auf den Prothesenkörper (1) verstellbar ist, um die Spreizung zwischen den nicht durchgehenden Teilen des Prothesenkörpers (1) einzustellen,
**dadurch gekennzeichnet, dass** die Längsachse der Öffnung (8) und der Justierungsvorrichtung (2) mit Bezug auf die zentrale Längsachse des Prothesenkörpers (1) geneigt ist, wobei die beiden Achsen sich im Allgemeinen in einer Sagittalebene des Prothesenkörpers (1) befinden.

2. Prothese gemäß Anspruch 1, wobei die Längsachse der Öffnung (8) und der Justierungsvorrichtung (2) mit Bezug auf die zentrale Längsachse des Prothesenkörpers (1) um 1 bis 15° geneigt ist, wobei die Neigung bevorzugt 1 ° bis 10° beträgt.

3. Prothese gemäß Anspruch 1, die eine ebene Längsseitenwand (11) umfasst, die durch den Spalt (10) in zwei Teile unterteilt ist.

4. Prothese gemäß Anspruch 1, die eine konkave Längsseitenwand (12) umfasst, die der ebenen Längsseitenwand (11) gegenüberliegt und ebenfalls durch den Spalt (10) in zwei Teile unterteilt ist.

5. Prothese gemäß Anspruch 1, die einen ebenen hinteren Teil (7) umfasst.

6. Prothese gemäß Anspruch 1, die einen abgerundeten vorderen Teil (9) umfasst.

7. Prothese gemäß Anspruch 1, wobei der Prothesenkörper (1) eine Struktur aufweist, die "wabenartig" durchbrochen ist.

8. Prothese gemäß Anspruch 1, wobei der obere Teil (3) und der untere Teil (6) in Längsrichtung angeordnete Öffnungen (5) und Querrillen (4) in Form von Zähnen aufweisen.

9. Prothese gemäß Anspruch 6, wobei der obere Teil (3) konvex und verformbar ist und an der Innenwölbung des oberhalb liegenden Wirbelkörpers auf der Sagittalebene und auf der Frontalebene anliegt.

10. Prothese gemäß Anspruch 1, wobei sich der Spalt (10) über mindestens die Hälfte der Länge des Prothesenkörpers (1) erstreckt.

11. Prothese gemäß Anspruch 1, wobei sich der Spalt (10) über mindestens zwei Drittel der Länge des Prothesenkörpers (1) erstreckt.

12. Prothese gemäß Anspruch 1, wobei der durchgehende Teil dem hinteren Teil (7) entspricht und dem vorderen Teil (9) gegenüberliegt.

13. Prothese gemäß Anspruch 1, wobei der untere Teil (6) nicht verformbar und eben ist.

14. Prothese gemäß Anspruch 1, wobei die Justierungsvorrichtung (2) hohl, von lang gestreckter, zylindrischer Form ist und einen ersten, als Anschlag dienenden Teil (13), einen zweiten, aus einer Gewindesteigung bestehenden Teil (14) und einen glatten dritten Teil (15), in Form einer Halbkugel (16) und mit durchgehenden Öffnungen (17), umfasst.

15. Prothese gemäß Anspruch 1, wobei es sich bei der Prothese um eine Prothese handelt, die sich mit Bezug auf eine Sagittalebene rechts oder links davon befindet.
